# EUROPEAN PATENT APPLICATION

(11) **EP 1 742 053 A1**
(43) Date of publication of application: **10.01.2007**
(21) Application number: 05709634.9
(22) Date of filing: 02.02.2005
(51) Int. Cl.: G01N 33/52, G01N 21/78

(54) **TEST PIECE FOR ANALYSIS**

(30) Priority: 16.04.2004 JP 2004122119
(71) Applicant: NGK INSULATORS, LTD., Nagoya-shi, Aichi 467-8530 (JP)
(72) Inventor: OHNISHI, Takao, Nagoya-shi, Aichi 4678530 (JP); HIROTA, Toshikazu, Nagoya-shi, Aichi 4678530 (JP)
(74) Representative: Paget, Hugh Charles Edward
(86) International application number: PCT/JP2005/001514
(87) International publication number: WO 2005/100988

(57) **Abstract**

The present invention provides an analytical test piece 10 including a carrier 1, and a reaction section 2 containing a detection component, the reaction section being provided on the surface and/or in the interior of the carrier 1, wherein in use, when a sample containing an analyte is introduced to the surface of the carrier 1, the analyte comes into contact and reacts with the detection component contained in the reaction section 2, to thereby generate a detectable substance (signal substance) or to exhibit a detectable property (signal property), and wherein the amount of the detection component contained in the reaction section 2 is continuously increased or decreased from one end (X) of the reaction section 2 to another end (Y) thereof. The analytical test piece exhibits high quality (e.g., high accuracy, high density, or high sensitivity), and facilitates accurate testing with only a small amount of a sample.

## Description

### Technical Field

The present invention relates to a test piece employed for analysis (hereinafter may be referred to as an "analytical test piece") which includes a reaction section containing a component for detecting a substance (hereinafter the component may be referred to as a "detection component"), wherein in use, when a sample containing an analyte is introduced to the test piece, the analyte contained in the sample comes into contact and reacts with the detection component contained in the reaction section, to thereby generate a detectable substance (signal substance) or to exhibit a detectable property (signal property). More particularly, the present invention relates to an analytical test piece which is useful as a test chip for testing or analyzing properties of a sample containing an analyte (e.g., a body fluid (in particular, urine or blood) of a human or an animal), which facilitates accurate testing with only a small amount of a sample, and which exhibits high quality (e.g., high accuracy, high density, or high sensitivity).

### Background Art

In relation to an analytical test piece for testing or analyzing properties of a sample containing an analyte (e.g., a body fluid (in particular, urine or blood) of a human or an animal); for example, an analytical test piece including a test section formed of a highly absorbable porous structure (e.g., porous layer or porous membrane) for causing a sample to be uniformly absorbed therein, there have been disclosed a porous membrane which prevents liquid communication between adjacent test sections, and a method for producing the porous membrane (see Patent Document 1). Also, there has been disclosed an analytical test piece including one or more test sections having a detection section for detecting a detectable substance, the detection section containing an inorganic layered compound (e.g., synthetic smectite) (see Patent Document 2).
Patent Document 1: Japanese Patent Application Laid-Open (kokai) No. H02-6541
Patent Document 2: Japanese Patent Application Laid-Open (kokai) No. H09-184837

However, the porous membrane, analytical test piece, and production method therefor disclosed in Patent Documents 1 and 2 pose problems in terms of poor stability due to rapid deterioration of detection components, and low analytical reliability due to low reaction efficiency, since a plurality of detection component species are contained in a single buffer in a reaction section. In addition, for example, the above-disclosed techniques encounter difficulty in meeting recent requirements for analytical test pieces (e.g., attainment of high accuracy, high density, and high sensitivity), and thus the techniques are not necessarily satisfactory.

In view of the foregoing, an object of the present invention is to provide an analytical test piece which is useful as a test chip for testing or analyzing properties of a sample containing an analyte (e.g., a body fluid (in particular, urine or blood) of a human or an animal), which facilitates accurate testing with only a small amount of a sample, and which exhibits high quality (e.g., high accuracy, high density, or high sensitivity).

### Disclosure of the Invention

In order to achieve the aforementioned object, the present invention provides an analytical test piece as described below.

[1] An analytical test piece comprising a carrier, and a reaction section containing a detection component, the reaction section being provided on the surface and/or in the interior of the carrier, wherein in use, when a sample containing an analyte is introduced to the surface of the carrier, the analyte comes into contact and reacts with the detection component contained in the reaction section, to thereby generate a detectable substance (signal substance) or to exhibit a detectable property (signal property), and wherein the amount of the detection component contained in the reaction section is continuously increased or decreased from one end of the reaction section to another end thereof (hereinafter the analytical test piece may be referred to as a "first invention").

[2] An analytical test piece as described in [1] above, wherein the detection component contained in the reaction section is formed of a plurality of species which are isolated or separated from one another on the reaction section, and the amount of each of the detection component species is continuously increased or decreased from one end of the reaction section to another end thereof.

[3] An analytical test piece as described in [1] or [2] above, wherein the detection component contained in the reaction section is in the form of an aggregate of spots (dots) which are provided in a predetermined arrangement pattern (spot pitch).

[4] An analytical test piece as described in [3] above, wherein the amount of the detection component contained in the spots (dots) constituting the arrangement pattern is continuously increased or decreased from one end of the reaction section to another end thereof.

[5] An analytical test piece as described in [3] or [4] above, wherein the spots (dots) constituting the arrangement pattern have an arrangement density which is continuously increased or decreased from one end of the reaction section to another end thereof.

[6] An analytical test piece as described in any of [3] to [5] above, wherein the size of the spots (dots) constituting the arrangement pattern is continuously increased or decreased from one end of the reaction section to another end thereof.

[7] An analytical test piece as described in any of [3] to [6] above, wherein the amount of the detection component contained in the spots (dots) constituting the arrangement pattern, the amount being measured in a thickness direction of the carrier, is continuously increased or decreased from one end of the reaction section to another end thereof.

[8] An analytical test piece comprising a carrier, and a reaction section containing a detection component, the reaction section being provided on the surface and/or in the interior of the carrier, wherein in use, when a sample containing an analyte is introduced to the surface of the carrier, the analyte comes into contact and reacts with the detection component contained in the reaction section, to thereby generate a detectable substance (signal substance) or to exhibit a detectable property (signal property), and wherein the reaction section is formed of a plurality of divided reaction sites, and the amount of the detection component contained in the reaction section is increased or decreased from one end of the reaction section to another end thereof in a stepwise manner when the reaction sites are adjacent to one another, or in a fragmentary manner when the reaction sites are isolated or separated from one another (hereinafter the analytical test piece may be referred to as a "second invention").

[9] An analytical test piece as described in [8] above, wherein the detection component contained in the reaction section is formed of a plurality of species which are isolated or separated from one another on the reaction section; and the amount of each of the detection component species, which respectively correspond to a plurality of the reaction sites, is increased or decreased from one end of the reaction section to another end thereof in a stepwise manner when the reaction sites are adjacent to one another, or in a fragmentary manner when the reaction sites are isolated or separated from one another.

[10] An analytical test piece as described in [8] or [9] above, wherein the detection component contained in the reaction section is in the form of an aggregate of spots (dots) which are provided in a predetermined arrangement pattern (spot pitch).

[11] An analytical test piece as described in [10] above, wherein the amount of the detection component contained in the spots (dots) constituting the arrangement pattern is increased or decreased, in a plurality of the divided reaction sites constituting the reaction section, from one end of the reaction section to another end thereof in a continuous, stepwise manner or in a discontinuous, fragmentary manner.

[12] An analytical test piece as described in [10] or [11] above, wherein the spots (dots) constituting the arrangement pattern have an arrangement density which is increased or decreased, in a plurality of the divided reaction sites constituting the reaction section, from one end of the reaction section to another end thereof in a continuous, stepwise manner or in a discontinuous, fragmentary manner.

[13] An analytical test piece as described in any of [10] to [12] above, wherein the size of the spots (dots) constituting the arrangement pattern is increased or decreased, in a plurality of the divided reaction sites constituting the reaction section, from one end of the reaction section to another end thereof in a continuous, stepwise manner or in a discontinuous, fragmentary manner.

[14] An analytical test piece as described in any of [10] to [13] above, wherein the amount of the detection component contained in the spots (dots) constituting the arrangement pattern, the amount being measured in a thickness direction of the carrier, is increased or decreased, in a plurality of the divided reaction sites constituting the reaction section, from one end of the reaction section to another end thereof in a continuous, stepwise manner or in a discontinuous, fragmentary manner.

[15] An analytical test piece as described in any of [1] to [14] above, wherein the carrier is a fibrous carrier or a porous carrier.

[16] An analytical test piece as described in any of [1] to [15] above, wherein the reaction section containing the detection component is provided on the surface and/or in the interior of the carrier through the ink-jet method.

The present invention provides an analytical test piece which is useful as a test chip for testing or analyzing properties of a sample containing an analyte (e.g., a body fluid (in particular, urine or blood) of a human or an animal), which facilitates accurate testing with only a small amount of a sample, and which exhibits high quality (e.g., high accuracy, high density, or high sensitivity).

### Brief Description of the Drawings

[Fig. 1] Fig. 1 is an explanatory view schematically showing a basic embodiment (first basic embodiment) of an analytical test piece according to the first invention.
[Fig. 2] Fig. 2 is an explanatory view schematically showing a modification (first modification 1) of the reaction section of the analytical test piece according to the first invention (first basic embodiment) shown in Fig. 1.
[Fig. 3] Fig. 3 is a partially enlarged view schematically showing a portion (portion A) of another modification (first modification 2) of the reaction section of the analytical test piece according to the first invention (first basic embodiment) shown in Fig. 1.
[Fig. 4] Fig. 4 is a partially enlarged view schematically showing a portion (portion A) of another modification (first modification 3) of the reaction section of the analytical test piece according to the first invention (first basic embodiment) shown in Fig. 1.
[Fig. 5] Fig. 5 is a partially enlarged view schematically showing a portion (portion A) of another modification (first modification 4) of the reaction section of the analytical test piece according to the first invention (first basic embodiment) shown in Fig. 1.
[Fig. 6] Fig. 6 is a partially enlarged view schematically showing a portion (portion A) of another modification (first modification 5) of the reaction section of the analytical test piece according to the first invention (first basic embodiment) shown in Fig. 1.
[Fig. 7(a)] Fig. 7(a) is a partially enlarged view schematically showing a portion (portion A) of another modification (first modification 6) of the reaction section of the analytical test piece according to the first invention (first basic embodiment) shown in Fig. 1.
[Fig. 7(b)] Fig. 7(b) is a cross-sectional view in a thickness direction of the carrier shown in Fig. 7(a), as taken along line A-A.
[Fig. 8(a)] Fig. 8(a) is an explanatory view schematically showing a basic embodiment (second basic embodiment 1) of an analytical test piece according to the second invention.
[Fig. 8(b)] Fig. 8(b) is a partially enlarged view showing the reaction section (portion B) of the analytical test piece according to the second invention (second basic embodiment 1) shown in Fig. 8(a).
[Fig. 9(a)] Fig. 9(a) is an explanatory view schematically showing another basic embodiment (second basic embodiment 2) of an analytical test piece according to the second invention. [Fig. 9(b)] Fig. 9(b) is a partially enlarged view showing the reaction section (portion D) of the analytical test piece according to the second invention (second basic embodiment 2) shown in Fig. 9(a).
[Fig. 10] Fig. 10 is an explanatory view schematically showing a modification (second modification 1) of the reaction section of the analytical test piece according to the second invention (second basic embodiment 1) shown in Fig. 8(a).
[Fig. 11] Fig. 11 is a partially enlarged view schematically showing a portion (portion C) of another modification (second modification 2) of the reaction section of the analytical test piece according to the second invention (second basic embodiment 1) shown in Fig. 8(a).
[Fig. 12] Fig. 12 is a partially enlarged view schematically showing another modification (second modification 3) of the reaction section (portion B) of the analytical test piece according to the second invention (second basic embodiment 1) shown in Fig. 8(a).
[Fig. 13] Fig. 13 is a partially enlarged view schematically showing another modification (second modification 4) of the reaction section (portion B) of the analytical test piece according to the second invention (second basic embodiment 1) shown in Fig. 8(a).
[Fig. 14] Fig. 14 is a partially enlarged view schematically showing another modification (second modification 5) of the reaction section (portion B) of the analytical test piece according to the second invention (second basic embodiment 1) shown in Fig. 8(a).
[Fig. 15(a)] Fig. 15(a) is a partially enlarged view schematically showing another modification (first modification 6) of the reaction section (portion B) of the analytical test piece according to the second invention (second basic embodiment 1) shown in Fig. 8(a).
[Fig. 15(b)] Fig. 15(b) is a cross-sectional view in a thickness direction of the carrier shown in Fig. 15(a), as taken along line C-C.

### Description of Reference Numerals

1. Carrier; 1a. Isolating portion; 2. Reaction section; 3. Spot (dot); 3a to 3m, 3o to 3s. Spot (dot); 4. Reaction site; 4a to 4s. Reaction site; 5. Reaction site; 5a to 5d. Reaction site; 10, 10a, 20, 20a. Analytical test piece; X (X(A), X(B)), R (R(A), R(B)). One end of reaction section; Y (Y(A), Y(B)), S (S(A), S(B)). Other end of reaction section

### Best Mode for Carrying Out the Invention

Best modes for carrying out the present invention will next be described in detail with reference to the drawings.

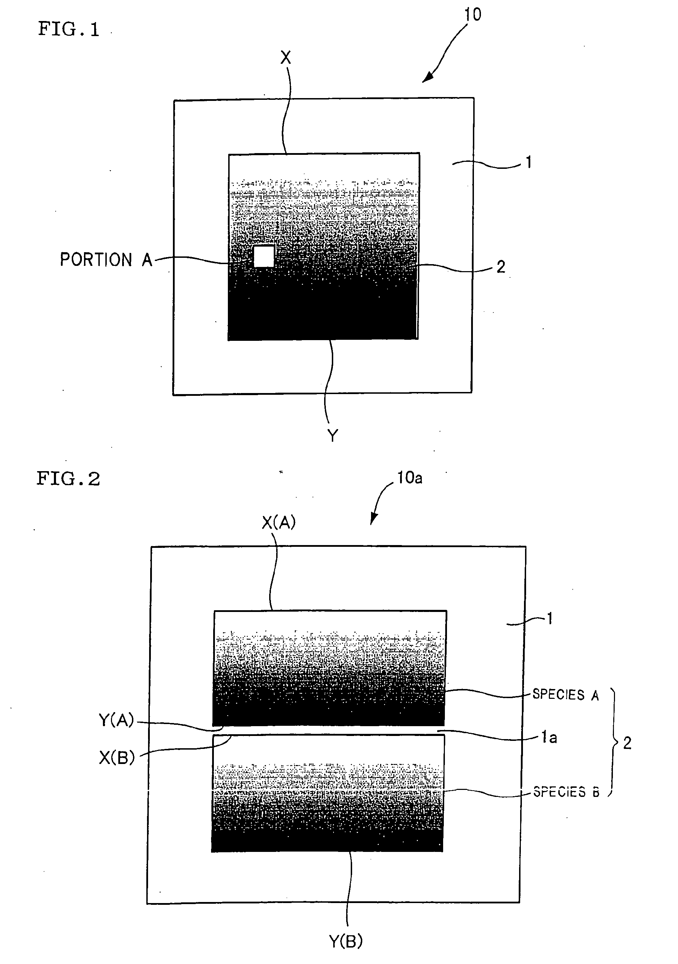

Fig. 1 is an explanatory view schematically showing a basic embodiment (first basic embodiment) of an analytical test piece according to the first invention. As shown in Fig. 1, the analytical test piece 10 according to the first invention includes a carrier 1, and a reaction section 2 containing a detection component, the reaction section being provided on the surface and/or in the interior of the carrier 1, wherein in use, when a sample containing an analyte (not illustrated) is introduced to the surface of the carrier 1, the analyte comes into contact and reacts with the detection component contained in the reaction section 2, to thereby generate a detectable substance (signal substance) or to exhibit a detectable property (signal property), and wherein the amount of the detection component contained in the reaction section 2 is continuously increased or decreased from one end (X) of the reaction section 2 to another end (Y) thereof. Fig. 1 shows the case where the amount of the detection component is continuously increased from one end (X) of the reaction section 2 to another end (Y) thereof.

Thus, when the amount of the detection component per unit area (volume) of the reaction section 2 is varied in the analytical test piece 10, the concentration of the analyte contained in the sample can be measured with a single analytical test piece in an analog manner, and the analyte concentration can be converted into numerical data. Therefore, merely a small amount of a sample required for a single analytical test piece can provide accurate test results.

No particular limitation is imposed on the carrier 1 employed in the first invention, so long as a detection component can be provided or held on the surface and/or in the interior of the carrier so as to form a reaction section. However, the carrier employed in the first invention is preferably a carrier having a flat surface. Examples of preferred carriers include a fibrous carrier and a porous carrier. Particularly, a hydrophilic fibrous or porous carrier is preferred. The material of such a carrier is preferably, for example, a cellulose, a polyethersulfone, or an acrylic polymer, and the pore diameter thereof is preferably 0.2 µm to several µm. Thus, when the carrier 1 is formed of a fibrous or porous carrier, an increased amount of the detection component, which constitutes the reaction section, can be permeated into the carrier 1; i.e., a large amount of the detection component can be held in a thickness direction of the carrier. Therefore, the resultant test piece exhibits increased analytical sensitivity.

No particular limitation is imposed on the detection component employed in the first invention, so long as the detection component can come into contact and react with an analyte contained in a sample to be introduced, to thereby generate a signal substance or to exhibit a signal property. The detection component may be formed of a single species or a combination of a plurality of species. In the case where the detection component is formed of a single species, when, for example, the activity of lactate dehydrogenase (i.e., an analyte) is measured; i.e., when a solution containing lactate dehydrogenase is employed as a sample, the detection component may be provided in the form of a solution containing lactic acid serving as a substrate, NAD (nicotinamide adenine dinucleotide) serving as a coenzyme, 1-methoxy PMS (phenazine methosulfate) serving as an electron carrier, and NTB (nitrotetrazolium blue) serving as a tetrazolium reagent, in which the pH is adjusted to a predetermined level by use of a buffer such as a phosphate buffer or a Tris-HCl buffer. The aforementioned solution may contain a predetermined amount of a surfactant. Addition of a surfactant to the solution enables the detection component to be uniformly dispersed in the surface/interior of the carrier. The surfactant to be added may be any surfactant, such as an anionic, cationic, or nonionic surfactant, and may be appropriately selected in consideration of conditions under which the surfactant is employed. Examples of preferred anionic surfactants include alkylallylsulfonic acid salts and alkylbenzenesulfonic acid salts; examples of preferred cationic surfactants include alkyltrimethylammonium and alkylpyridinium; and examples of preferred nonionic surfactants include polyoxyethylene fatty acid esters and polyoxyethylene alkylphenyl.

In the first invention, preferably, the detection component contains a color-developing substance. In the case where a plurality of detection component species are employed in combination, preferably, at least one of the component species contains a color-developing substance. Incorporation of a color-developing substance into the detection component enables signal substance generation or signal property to be exhibited clearly. Examples of the solution containing such a color-developing substance include a solution containing a reducing substance (e.g., a tetrazolium reagent) and a solution containing an oxidizing substance (e.g., 4-aminoantipyrine or phenol). In the case where a plurality of detection component species are employed in combination, preferably, the detection component species contain color-developing substances which develop different colors. Incorporation of such different color-developing substances facilitates easy and accurate assessment of test results through visual inspection.

Fig. 2 is an explanatory view schematically showing a modification (first modification 1) of the reaction section of the analytical test piece according to the first invention (first basic embodiment) shown in Fig. 1. As shown in Fig. 2, the first modification 1 is configured such that the detection component contained in a reaction section 2 of an analytical test piece 10a is formed of a plurality of species which are isolated or separated from each other on the reaction section, and the amount of each of the detection component species is continuously increased or decreased from one end (X(A) or X(B)) of the reaction section 2 to another end (Y(A) or Y(B)) thereof. Fig. 2 shows the case where the detection component is formed of two species (species A and B) which are isolated or separated from each other on the reaction section. Fig. 2 shows the case where the amount of the detection component species A (or species B) is increased toward another end Y(A) (or Y(B)) (i.e., on the lower side as viewed in Fig. 2). Fig. 2 shows the case where the two detection component species (species A and B) are isolated or separated from each other by an isolating portion 1a.

With this configuration, merely a small amount of a sample enables a plurality of items to be tested accurately.

Examples of combinations of such two detection component species (species A and B) isolated or separated from each other include a combination of a first detection component species (species A) for the analysis of glucose and a second detection component species (species B) for the analysis of cholesterol.

Specific examples of the aforementioned combination include a combination of the detection component species A in the form of a solution containing glucose dehydrogenase, NAD (nicotinamide adenine dinucleotide) serving as a coenzyme, diaphorase serving as an electron carrier, and MTT (3-(4,5-dimethylthiazol-2-yl)-2,5-diphenyl-2H-tetrazolium bromide), and the detection component species B in the form of a solution containing cholesterol dehydrogenase, NAD (nicotinamide adenine dinucleotide) serving as a coenzyme, 1-methoxy PMS (phenazine methosulfate) serving as an electron carrier, and NTB (nitrotetrazolium blue) serving as a tetrazolium reagent, in which the pH is adjusted to a predetermined level by use, for example, of a Tris-HCl buffer. Any of the aforementioned solutions may contain a predetermined amount of a surfactant. Addition of a surfactant to the solution enables the detection component to be uniformly dispersed in the surface/interior of the carrier. The surfactant to be added may be any surfactant, such as an anionic, cationic, or nonionic surfactant, and may be appropriately selected in consideration of conditions under which the surfactant is employed. Examples of preferred anionic surfactants include alkylallylsulfonic acid salts and alkylbenzenesulfonic acid salts; examples of preferred cationic surfactants include alkyltrimethylammonium and alkylpyridinium; and examples of preferred nonionic surfactants include polyoxyethylene fatty acid esters and polyoxyethylene alkylphenyl. In the aforementioned configuration, when the detection component species A detects glucose, a blue color is developed, whereas when the detection component species B detects cholesterol, a red-violet color is developed.

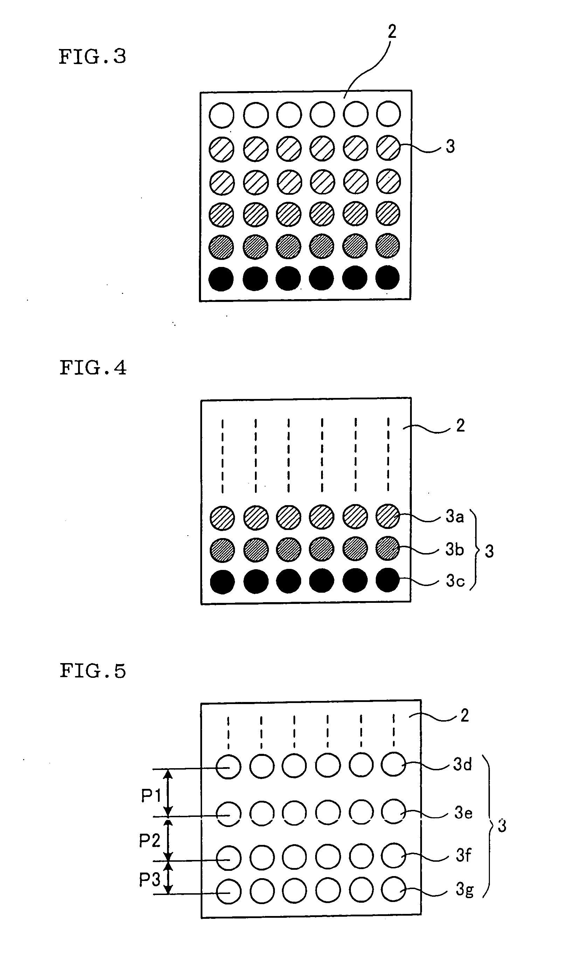

Fig. 3 is a partially enlarged view schematically showing a portion (portion A) of another modification (first modification 2) of the reaction section of the analytical test piece according to the first invention (first basic embodiment) shown in Fig. 1. As shown in Fig. 3, the first modification 2 is configured such that the detection component contained in a reaction section 2 is in the form of an aggregate of spots (dots) 3 which are provided in a predetermined arrangement pattern (spot pitch). Fig. 3 shows the case where the amount of detection component in the form of the spots (dots) 3 is increased toward another end (Y) of Fig. 1 (i.e., on the lower side as viewed in Fig. 3). In this case, no particular limitation is imposed on the shape of the spots (dots) 3, and the spots (dots) may have, for example, a circular shape, an elliptical shape, an elongated circular shape, or a racing-track-like shape as viewed in a predetermined plane (cross section) which is in parallel with the surfaces of the carrier 1 (e.g., in a plane at a half-depth position of the carrier 1).

With this configuration, a sample can be readily supplied to the detection component provided on the surface or in the interior of the carrier from the surrounding of the detection component, and an analyte contained in the sample can be efficiently reacted with the thus-provided detection component. Therefore, merely a small amount of the detection component provides sufficient detection sensitivity. In addition, since reaction can proceed through the entirety of the surface of the detection component dots, the time required for detection can be reduced.

Methods for forming spots (dots) and an arrangement pattern will be described below.

Fig. 4 is a partially enlarged view schematically showing a portion (portion A) of another modification (first modification 3) of the reaction section of the analytical test piece according to the first invention (first basic embodiment) shown in Fig. 1. As shown in Fig. 4, the first modification 3 is configured such that the amount of the detection component which is in the form of spots (dots) 3 constituting an arrangement pattern of a reaction section 2 is continuously increased or decreased from one end (X) of the reaction section 2 to another end (Y) thereof. Fig. 4 shows, among the spots (dots) 3, three types of spots (dots) 3a, 3b, and 3c having different amount of the detection component, in which the detection component content is lowest in the spots (dots) 3a, and is highest in the spots (dots) 3c.

With this configuration, sensitivity can be varied without changing the dot size. Therefore, the resultant analytical test piece exhibits high dynamic range with small area.

Fig. 5 is a partially enlarged view schematically showing a portion (portion A) of another modification (first modification 4) of the reaction section of the analytical test piece according to the first invention (first basic embodiment) shown in Fig. 1. As shown in Fig. 5, the first modification 4 is configured such that the arrangement density of spots (dots) 3 constituting an arrangement pattern of a reaction section 2 is continuously increased or decreased from one end (X) of the reaction section 2 to another end (Y) thereof (see Fig. 1). Fig. 5 shows, among the spots (dots) 3, four types of spots (dots) 3d, 3e, 3f, and 3g which are arranged at different arrangement densities, in which the distance (pitch) p1 between the spots 3d and 3e, the distance (pitch) p2 between the spots 3e and 3f, and the distance (pitch) p3 between the spots 3f and 3g are decreased toward another end (Y) (i.e., p1 > p2 > p3). The distance (pitch) between spots may be varied in a direction perpendicular to the direction from one end (X) to another end (Y), or both in the direction from one end (X) to another end (Y) and in a direction perpendicular to the (X)-(Y) direction.

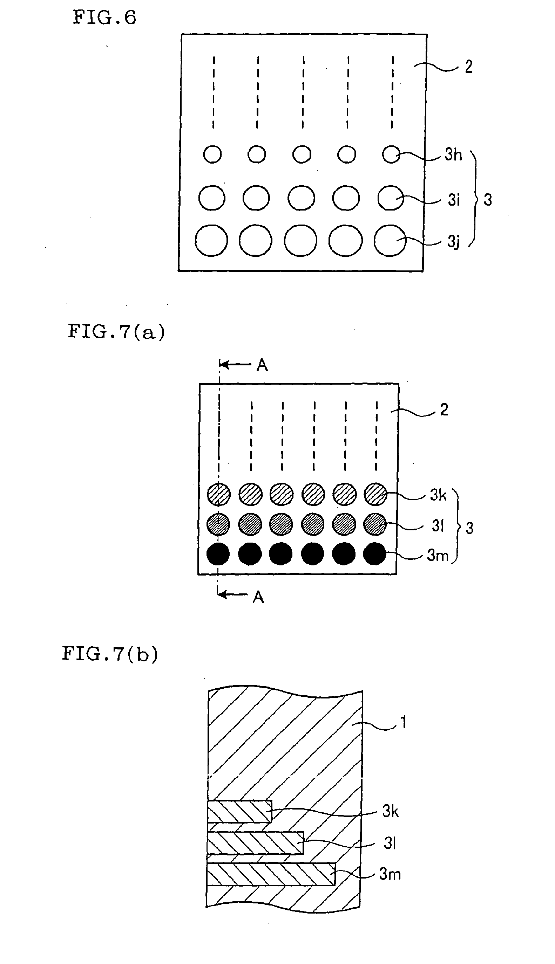

Fig. 6 is a partially enlarged view schematically showing a portion (portion A) of another modification (first modification 5) of the reaction section of the analytical test piece according to the first invention (first basic embodiment) shown in Fig. 1. As shown in Fig. 6, the first modification 5 is configured such that the size of spots (dots) 3 constituting an arrangement pattern of a reaction section 2 is continuously increased or decreased from one end (X) of the reaction section 2 to another end (Y) thereof (see Fig. 1). Fig. 6 shows, among the spots (dots) 3, three types of spots (dots) 3h, 3i, and 3j having different sizes, in which the size of the spots (dots) is increased toward another end (Y) (the size of the spots (dots) 3h is smallest, and the size of the spots (dots) 3j is largest).

With the above-described configuration of the first modification 4 or 5, sensitivity can be readily varied by use of a detection component (solution) of single concentration (or content), and therefore the analytical test piece can be produced at high productivity. In addition, sensitivity can be reliably varied in a single analytical test piece.

Fig. 7(a) is a partially enlarged view schematically showing a portion (portion A) of another modification (first modification 6) of the reaction section of the analytical test piece according to the first invention (first basic embodiment) shown in Fig. 1. Fig. 7(b) is a cross-sectional view in a thickness direction of the carrier shown in Fig. 7(a), as taken along line A-A. As shown in Fig. 7(a), the first modification 6 is configured such that the amount of the detection component which is in the form of spots (dots) 3 constituting an arrangement pattern of a reaction section 2, the amount being measured in a thickness direction of a carrier 1, is continuously increased or decreased from one end (X) of the reaction section 2 to another end (Y) thereof (see Fig. 1). Fig. 7(a) shows, among the spots (dots) 3, three types of spots (dots) 3k, 31, and 3m having different amount of the detection componentas measured in a thickness direction of the carrier 1, in which the detection component content is increased toward another end (Y) (the detection component content is lowest in the spots (dots) 3k, and is highest in the spots (dots) 3m).

With this configuration, reaction is allowed to proceed in a thickness direction of the carrier 1. Therefore, the resultant analytical test piece exhibits high dynamic range with small area.

The first invention may include an appropriate combination of the aforementioned first basic embodiment and first modifications 1 to 6.

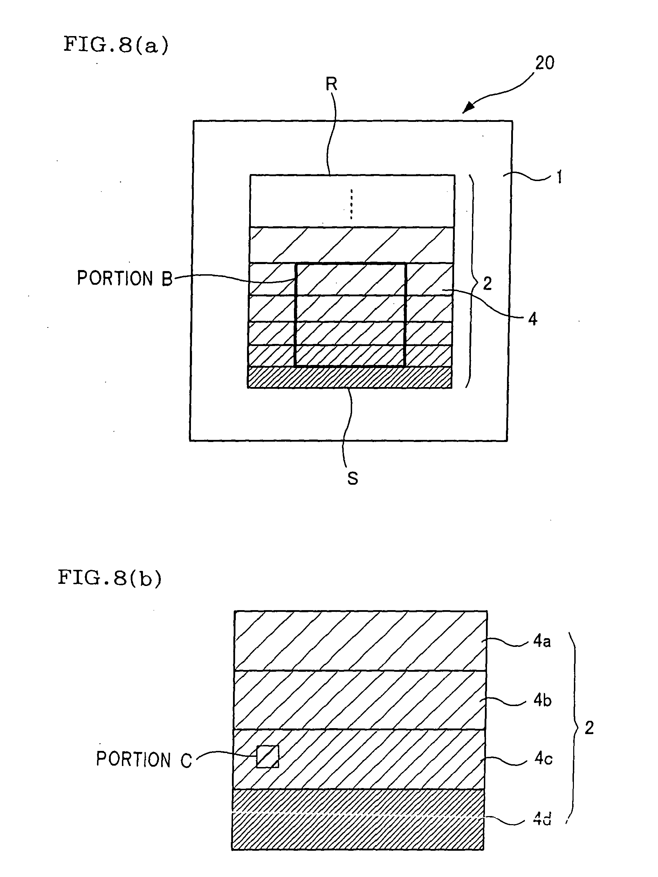

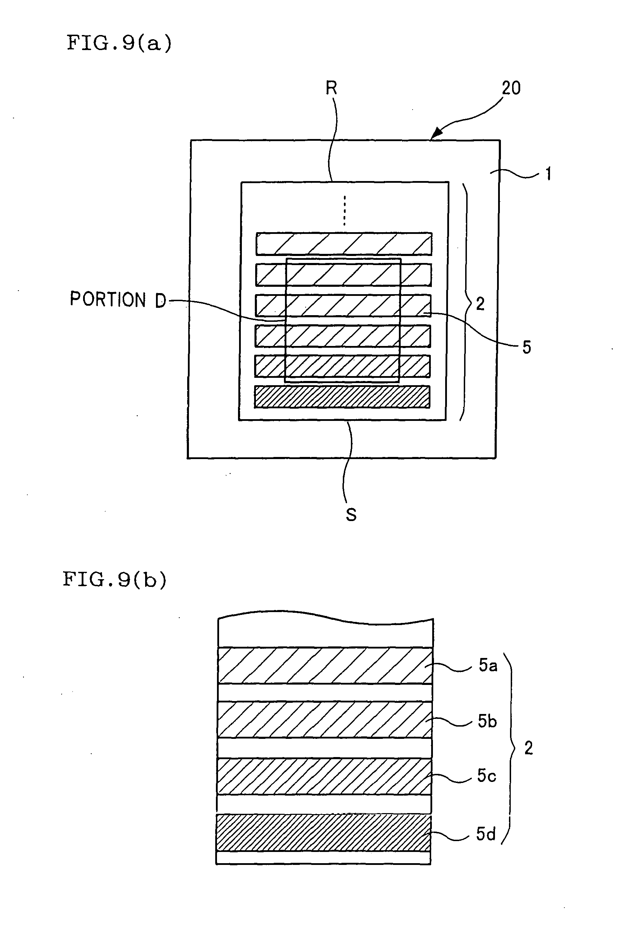

Fig. 8(a) is an explanatory view schematically showing a basic embodiment (second basic embodiment 1) of an analytical test piece according to the second invention; and Fig. 8(b) is a partially enlarged view showing the reaction section (portion B) of the analytical test piece according to the second invention (second basic embodiment 1) shown in Fig. 8(a). Fig. 9(a) is an explanatory view schematically showing another basic embodiment (second basic embodiment 2) of an analytical test piece according to the second invention; and Fig. 9(b) is a partially enlarged view showing the reaction section (portion D) of the analytical test piece according to the second invention (second basic embodiment 2) shown in Fig. 9(a). As shown in Figs. 8(a) and 9(a), the analytical test piece 20 according to the second invention includes a carrier 1, and a reaction section 2 containing a detection component, the reaction section being provided on the surface and/or in the interior of the carrier 1, wherein in use, when a sample containing an analyte is introduced to the surface of the carrier 1, the analyte comes into contact and reacts with the detection component contained in the reaction section 2, to thereby generate a detectable substance (signal substance) or to exhibit a detectable property (signal property). The reaction section 2 is formed of a plurality of divided reaction sites 4 or 5 (i.e., reaction sites 4a, 4b, 4c, and 4d shown in Fig. 8(b), or reaction sites 5a, 5b, 5c, and 5d shown in Fig. 9(b)), and the amount of the detection component contained in the reaction section 2 is increased or decreased from one end (R) of the reaction section 2 to another end (S) thereof in a stepwise manner when the reaction sites are adjacent to one another as shown in Fig. 8(a) (see the reaction sites 4a, 4b, 4c, and 4d of Fig. 8(b)), or in a fragmentary manner when the reaction sites are isolated or separated from one another as shown in Fig. 9(a) (see the reaction sites 5a, 5b, 5c, and 5d of Fig. 9(b)). As used herein, the expression "reaction sites are adjacent to one another" refers to the case where reaction sites are provided such that the outlines of the reaction sites are in contact with one another, as well as the case where reaction sites are provided at some intervals.

Thus, when the amount of the detection component per unit area (volume) of the reaction section 2 is varied in the analytical test piece 20, the concentration of the analyte contained in the sample can be measured with a single analytical test piece in an analog manner, and the analyte concentration can be converted into numerical data. Therefore, merely a small amount of a sample required for a single analytical test piece can provide accurate test results. When the area (volume) of a reaction site is increased; i.e., when a region containing a same amount of the detection component is increased, determination through visual inspection can be readily performed with accuracy. Specifically, when the width of a region containing a same amount of the detection component is regulated to 0.05 mm to 5 mm (more preferably 0.3 mm to 2 mm), determination through visual inspection is readily performed. When test is performed by means of a testing apparatus such as a laser beam apparatus, the size (width) of such a region may be appropriately regulated in accordance with the testing area of the apparatus. In the case of the second basic embodiment 2, since the amount of the detection component contained in the reaction section 2 is varied (increased or decreased) in a fragmentary manner in the reaction sites which are isolated or separated from one another, test can be readily performed automatically by means of a testing apparatus such as a laser beam apparatus. The size of discontinuous reaction sites may be appropriately varied in the analytical test piece for alignment, whereby automatic test can be readily performed.

The second invention may employ elements (e.g., carrier 1 and detection component) similar to those employed in the first invention, and may have a configuration similar to that of the first invention. Unless otherwise specified, the below-described modifications (second modifications) of the second invention correspond to the case where the amount of a detection component is increased or decreased in a stepwise manner in reaction sites which are adjacent to one another (i.e., the case shown in Fig. 8(a)).

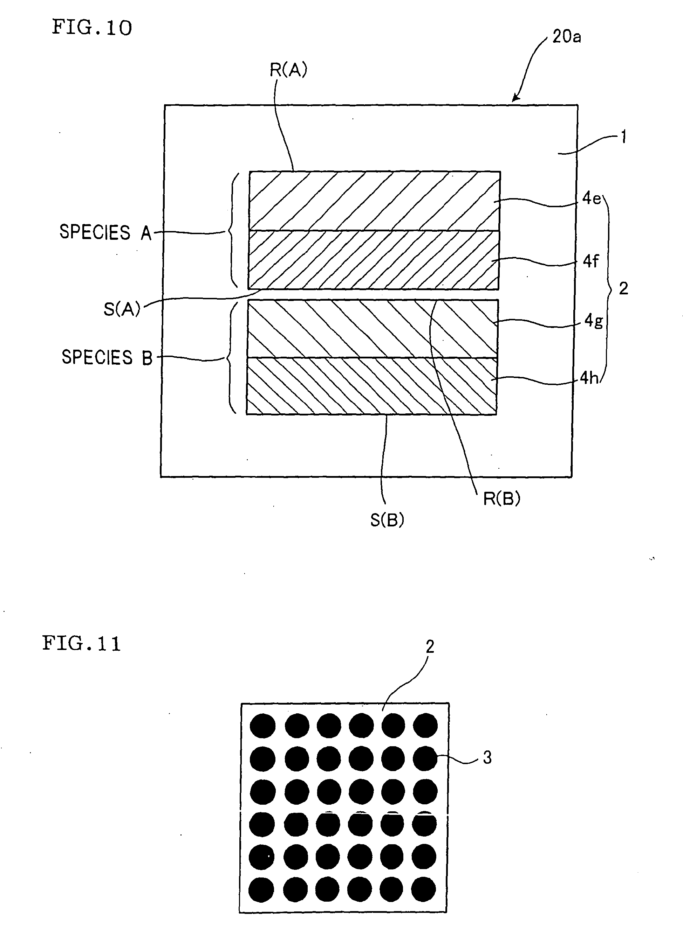

Fig. 10 is an explanatory view schematically showing a modification (second modification 1) of the reaction section of the analytical test piece according to the second invention (second basic embodiment 1) shown in Fig. 8(a). As shown in Fig. 10, the second modification 1 is configured such that the detection component contained in a reaction section 2 is formed of a plurality of species which are isolated or separated from each other on the reaction section (a region containing no test component is provided between the detection component species), and the amount of each of the detection component species, which respectively correspond to a plurality of reaction sites that are adjacent to each other, is increased or decreased from one end (R(A) or R(B)) of the reaction section to another end (S(A) or S(B)) thereof in a stepwise manner. Fig. 10 shows the case where the reaction section is formed of four reaction sites containing two detection component species (species A and B) which are separated from each other (i.e., reaction sites 4e and 4f which contain the detection component species A and are adjacent to each other, and reaction sites 4g and 4h which contain the detection component species B and are adjacent to each other). Fig. 10 shows the case where the detection component species A content of the reaction site 4f (on another end S(A) side) is greater than that of the reaction site 4e, and the detection component species B content of the reaction site 4h (on another end S(B) side) is greater than that of the reaction site 4g.

With this configuration, merely a small amount of a sample enables a plurality of items to be tested accurately.

Fig. 11 is a partially enlarged view schematically showing a portion (portion C) of another modification (second modification 2) of the reaction section 2 of the analytical test piece according to the second invention (second basic embodiment 1) shown in Fig. 8(a). As shown in Fig. 11, the second modification 1 is configured such that the detection component contained in a reaction section 2 is in the form of an aggregate of spots (dots) 3 which are provided in a predetermined arrangement pattern (spot pitch).

With this configuration, similar to the case of the first modification 2, a sample can be readily supplied to the detection component provided on the surface or in the interior of the carrier from the surrounding of the detection component, and an analyte contained in the sample can be efficiently reacted with the thus-provided detection component. Therefore, merely a small amount of the detection component provides sufficient detection sensitivity. In addition, since reaction can proceed through the entirety of the surface of the detection component dots, the time required for detection can be reduced.

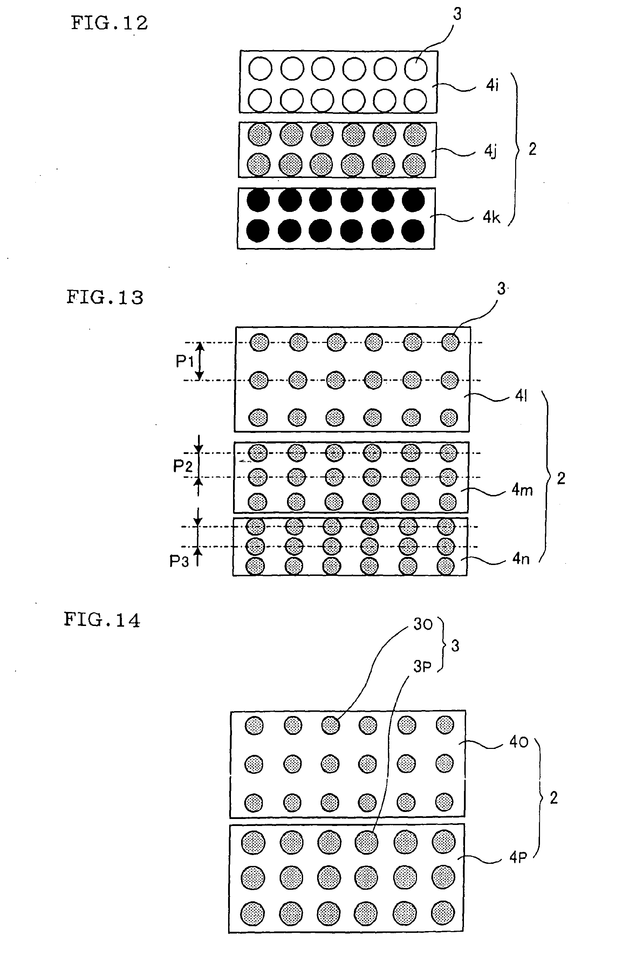

Fig. 12 is a partially enlarged view schematically showing another modification (second modification 3) of the reaction section (portion B) of the analytical test piece according to the second invention (second basic embodiment 1) shown in Fig. 8(a). As shown in Fig. 12, the second modification 3 is configured such that the amount of the detection component which is in the form of spots (dots) 3 constituting an arrangement pattern of a reaction section 2 is increased or decreased from one end (R) of the reaction section 2 to another end (S) thereof (see Fig. 8(a)) in a stepwise manner through a plurality of divided reaction sites 4 which are adjacent to one another and constitute the reaction section 2. Fig. 12 shows three adjacent reaction sites 4i, 4j, and 4k including spots (dots) 3 having different amount of the detection component, in which the detection component content is lowest in the reaction site 4i, and is highest in the reaction site 4k.

With this configuration, sensitivity can be varied without changing the dot size. Therefore, the resultant analytical test piece exhibits high dynamic range with small area.

Fig. 13 is a partially enlarged view schematically showing another modification (second modification 4) of the reaction section (portion B) of the analytical test piece according to the second invention (second basic embodiment 1) shown in Fig. 8(a). As shown in Fig. 13, the second modification 4 is configured such that the arrangement density of spots (dots) 3 constituting an arrangement pattern of a reaction section 2 is increased or decreased from one end (R) of the reaction section 2 to another end (S) thereof (see Fig. 8(a)) in a stepwise manner through a plurality of divided reaction sites 4 which are adjacent to one another and constitute the reaction section 2. Fig. 13 shows three adjacent reaction sites 41, 4m, and 4n including spots (dots) 3 which are arranged at different arrangement densities, in which the distance (pitch) p1 between the spots 3 in the reaction site 41, the distance (pitch) p2 between the spots 3 in the reaction site 4m, and the distance (pitch) p3 between the spots 3 in the reaction site 4n are decreased toward another end (S) (i.e., p1 > p2 > p3). The distance (pitch) between spots may be varied in a direction perpendicular to the direction from one end (R) to another end (S), or both in the direction from one end (R) to another end (S) and in a direction perpendicular to the (R)-(S) direction.

Fig. 14 is a partially enlarged view schematically showing another modification (second modification 5) of the reaction section (portion B) of the analytical test piece according to the second invention (second basic embodiment 1) shown in Fig. 8(a). As shown in Fig. 14, the second modification 5 is configured such that the size of spots (dots) 3 constituting an arrangement pattern of a reaction section 2 is increased or decreased from one end (R) of the reaction section 2 to another end (S) thereof (see Fig. 8(a)) in a stepwise manner through a plurality of divided reaction sites 4 which are adjacent to one another and constitute the reaction section 2. Fig. 14 shows two adjacent reaction sites 4o and 4p including spots (dots) 3 having different sizes, in which the size of spots (dots) 3o in the reaction site 4o is smaller than that of spots (dots) 3p in the reaction site 4p.

With this configuration, similar to the case of the first modification 4 or 5, sensitivity can be readily varied by use of a detection component (solution) of single concentration (or content), and therefore the analytical test piece can be produced at high productivity. In addition, sensitivity can be reliably varied in a single analytical test piece. The distance (pitch) between dots and the diameter of the dots may be appropriately determined in consideration of, for example, solution sensitivity or wettability of the carrier to a solution, so long as the dots are not in contact with one another. However, preferably, in order to facilitate visual inspection, the dot interval (i.e., the value obtained by subtracting the dot diameter from the pitch) is decreased to a possible small value within such a range that dots are not clearly visible to the naked eye. Specifically, the dot interval is preferably regulated to 0.5 mm or less, more preferably 0.1 mm or less.

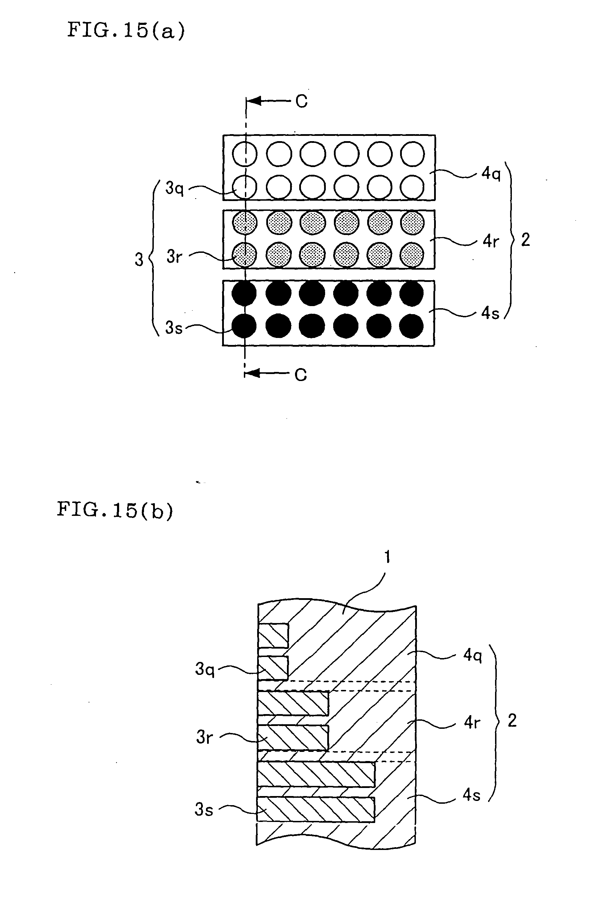

Fig. 15(a) is a partially enlarged view schematically showing another modification (second modification 6) of the reaction section (portion B) of the analytical test piece according to the second invention (second basic embodiment 1) shown in Fig. 8(a). Fig. 15(b) is a cross-sectional view in a thickness direction of the carrier shown in Fig. 15(a), as taken along line C-C. As shown in Fig. 15(a), the second modification 6 is configured such that the amount of the detection component which is in the form of spots (dots) 3 constituting an arrangement pattern of a reaction section 2, the amount being measured in a thickness direction of a carrier 1, is increased or decreased from one end (R) of the reaction section to another end (S) thereof (see Fig. 8(a)) in a stepwise manner through a plurality of divided reaction sites 4 which are adjacent to one another and constitute the reaction section 2. Fig. 15(a) shows three adjacent reaction sites 4q, 4r, and 4s including three types of spots (dots) 3q, 3r, and 3s having different detection component contents as measured in a thickness direction of the carrier 1, in which the detection component content is increased toward another end (S) (the detection component content is lowest in the reaction site 4q, and is highest in the reaction site 4s).

With this configuration, reaction is allowed to proceed in a thickness direction of the carrier 1. Therefore, the resultant analytical test piece exhibits high dynamic range with small area.

The second invention may include an appropriate combination of the aforementioned second basic embodiments 1 and 2 and second modifications 1 to 6. When a reaction section containing a plurality of separated detection component species is provided in a carrier in a thickness direction, and a substrate which prevents permeation of a solution (which selectively prevents permeation of an element of detection component) is provided between the different detection component species, a reagent must be added dropwise (applied) to both surfaces of the carrier, but different types of analytes can be readily and reliably tested in a small area.

In the present invention (the first invention and the second invention), preferably, the reaction section 2 containing the detection component is provided on the surface and/or in the interior of the carrier 1 through the ink-jet method.

For the production of an analytical test piece, generally, a reaction section containing detection-component is formed on a carrier through, for example, the impregnation method, the QUILL method, the pin and ring method, or the spring pin method.

In the impregnation method, a carrier is impregnated with a detection component (solution) so that the detection component (solution) is provided on the surface and/or in the interior of the carrier, followed by drying, to thereby immobilize the detection component to the carrier. However, this method poses problems in that, for example, the method encounters difficulty in meeting recent requirements for analytical test pieces (e.g., attainment of high accuracy, high density, and high sensitivity; specifically, formation of a reaction section containing detection components of different concentrations or densities).

In the QUILL method, a detection component (solution) is placed in a depression formed on a pin tip, and the pin tip is brought into contact with a carrier, to thereby transfer the detection component (solution) in the depression onto the carrier for formation of a spot (reaction section). However, this method poses problems in terms of, for example, durability (e.g., deformation or damage of a pin tip which would otherwise be caused by contact between the pin tip and a carrier), and frequent cross-contamination due to incomplete cleaning of a detection component (solution) from a depression of a pin tip.

In the pin and ring method, a detection component (solution) contained in a microplate is reserved in a ring, and subsequently a pin tip is penetrated inside the detection-component (solution)-reserved ring so that the pin tip captures the detection component (solution) reserved in the ring, followed by formation of a spot (reaction section) on a carrier. However, in this method, the number of detection components (solutions) which can be reserved at a time depends on the number of rings (conventionally, the number of such detection components has been limited to about several species), and therefore formation of microspots (reaction sections) of several thousands to several tens of thousands of detection component (solution) species requires several hundreds to several thousands of washing and drying steps. Thus, this method poses a problem in terms of low productivity. This method also poses a problem in terms of no reproducibility in permeation (amount) of a detection component (solution) in a carrier.

In the spring pin method, a detection component (solution) deposited on a pin tip is transferred onto a carrier through pressing of the pin tip onto the carrier, to thereby form a microspot (reaction section). This method employs a spring-incorporated dual-pin structure which ejects a detection component (solution) with less damage to a pin or a carrier. However, in this method, basically, only one spotting step is performed at one reserving step (i.e., low productivity). In all the aforementioned conventional microspot (reaction section) formation methods, a detection component (solution) is transferred onto a carrier while the detection component (solution) is exposed to air, and thus the detection section (solution) could be dried during the course of transfer, resulting in failure to perform spotting. Thus, any of these methods poses a problem in terms of low use efficiency of a very expensive detection component (solution).

In contrast, when the ink-jet method is employed for providing a reaction section 2 on the surface and/or in the interior of a carrier 1, a solution containing a detection component can be supplied to the surface, etc. of the carrier 1 in a non-contact manner, and an analytical test piece can be produced at high efficiency without causing damage, etc. to the carrier 1.

Employment of the ink-jet method can attain high sensitivity, high density, and high accuracy of the position of a reaction section (e.g., spots (dots) constituting a predetermined arrangement pattern), and can accurately regulate the amount of a solution to be added per spot (spot amount). Therefore, sensitivity can be accurately regulated to a predetermined level in an analytical test piece; analytical reliability can be improved; uniform quality can be maintained in an analytical test piece of large size; and production efficiency can be enhanced. Attainment of high density facilitates production of a highly sensitive analytical test piece which can detect only a small amount of a sample. In addition, since the amount of a solution to be added can be accurately regulated, error in measurements obtained by means of an analytical test piece can be reduced, and variation in measurements can be reduced.

The ink-jet method can employ, for example, a liquid droplet ejection unit including a flow path substrate on which a flow path is formed; an actuator section which is mounted on the flow path substrate and serves as a pressurizing chamber for varying the inner volume of a cavity; a nozzle substrate which is attached to the lower surface of the flow path substrate and is provided with an ejection nozzle; and a liquid injection section provided on the upper surface of a rear portion of the flow path substrate (hereinafter such a unit may be referred to as an "ejection unit"). Specifically, the ink-jet method preferably employs one or more ejection units described in Japanese Patent Application Laid-Open (kokai) No. 2003-75305.

In the case where a plurality of ejection units are employed, when a plurality of dots are formed simultaneously, an analytical test piece can be produced at high efficiency. When the sizes of ejection nozzles are varied, the amounts of detection components ejected through the ejection nozzles can be readily differentiated, which is preferred. The amounts of detection components to be ejected may be differentiated by varying, in the ejection units, the amount or rate of change in the inner volume of the cavity. Preferably, the amounts of detection components to be ejected from the ejection units are differentiated by varying, in the ejection units, the difference between the liquid level of the liquid injection section and the level of the ejection nozzle.

Such a liquid droplet ejection unit is preferably controlled by means of, for example, a system described in Japanese Patent Application Laid-Open (kokai) No. 2003-98183. In addition to the ink-jet method, there may be appropriately employed, for example, a screen printing technique in which mesh opening size is varied in consideration of the viscosity of a solution.

In the present invention, a support for supporting a carrier may be provided on the back surface (i.e., the surface opposite the front surface) of the carrier. Provision of such a support facilitates easy handling, and easy alignment during the course of formation of spots (dots). Examples of the material of such a support include metal, ceramic, glass, and resin.

On the outside of a reaction section formed on a carrier, an additional reaction section may be provided for confirming in advance whether or not a detection component reacts normally. In order to facilitate determination through visual inspection, a marking for showing reaction sensitivity may be provided in a plurality of divided reaction sites 4 constituting a reaction section 2 which is configured such that the amount of a detection component is increased or decreased from one end ((X) or (R)) of the reaction section to another end ((Y) or (S)) thereof in a continuous, stepwise manner or in a discontinuous, fragmentary manner. Such a marking may be, for example, a region containing no detection component.

### Examples

The present invention will next be described in more detail by way of Examples, which should not be construed as limiting the invention thereto. In Examples, an analytical test piece was produced through the ink-jet method for examining whether or not a single test solution can be detected in an analog manner.

### (Example 1)

There was employed a carrier formed of a hydrophilic-cellulose-mixed ester (size: 5 mm × 5 mm, pore diameter: 0.8 µm, thickness: 0.16 mm). On the carrier was provided a detection-component-containing solution; i.e., a solution containing 100 units/mL of glucose dehydrogenase, 20 mM β-NAD (nicotinamide adenine dinucleotide), 20 units/mL of diaphorase, and 20 mM MTT (3-(4,5-dimethylthiazol-2-yl)-2,5-diphenyl-2H-tetrazolium bromide). A reaction section was formed by means of the ejection unit (ink-jet method) described in Japanese Patent Application Laid-Open (kokai) No. 2003-75305, to thereby produce an analytical test piece sample for evaluation (size: 5 mm x 5 mm). In this evaluation sample, which includes eight reaction sites (i.e., eight sensitivity steps in an analog manner) in a region of 3 mm x 3 mm, the amount of the detection component (the amount of the solution) per unit area of each of the reaction sites constituting the reaction section was continuously increased from 1 nL/mm² to 8 nL/mm² in eight steps. An analyte-containing sample; i.e., a solution containing glucose (glucose content (parameter): 10 mg/dL, 20 mg/dL, or 50 mg/dL), was prepared, and the thus-prepared solution was applied onto the surface of the carrier of the evaluation sample. Sensitivity properties of the evaluation sample were evaluated through visual inspection. The results are shown in Table 1. In the reaction section of the evaluation sample, development of a blue color, which is as shown in Table 1, was observed in the reaction sites (having different detection component contents (solution contents)). In Table 1, symbol "x" corresponds to the case where no blue color development was observed; symbol "Δ" corresponds to the case where some blue color development was observed; and symbol "O" corresponds to the case where blue color development was observed.

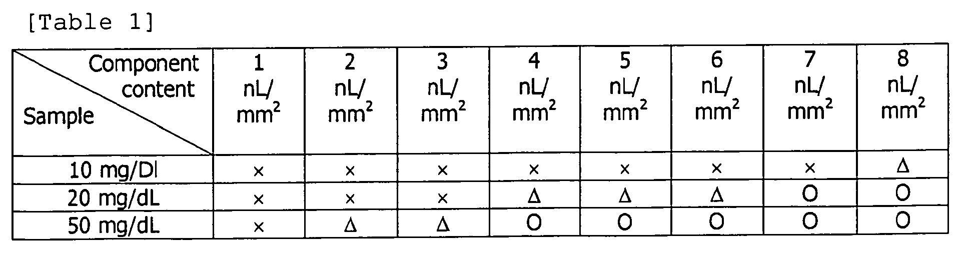

As is clear from Table 1, in one analytical test piece (one analytical test piece which is configured such that the detection component content of the reaction sites is continuously increased from one end of the reaction section to another end thereof), the glucose content is measured in an analog manner.

### (Example 2)

The procedure of Example 1 was repeated, except that the detection-component-containing solution to be provided on the carrier was replaced by a solution prepared by dissolving 10 units/mL of cholesterol dehydrogenase, 5 mM NAD (nicotinamide adenine dinucleotide), 20 units/mL of diaphorase, and 5 mM NTB in a Tris buffer (pH 8.0), and that the analyte-containing sample was replaced by a solution containing cholesterol (cholesterol content (parameter): 20 mg/dL, 40 mg/dL, or 100 mg/dL), to thereby produce a sample for evaluation. In a manner similar to that of Example 1, sensitivity properties of the evaluation sample were evaluated through visual inspection. The results are shown in Table 2. In the reaction section of the evaluation sample, development of a red-violet color, which is as shown in Table 2 (symbols in Table 2 have the same meanings as in Table 1), was observed in the reaction sites (having different detection component contents (solution contents)).

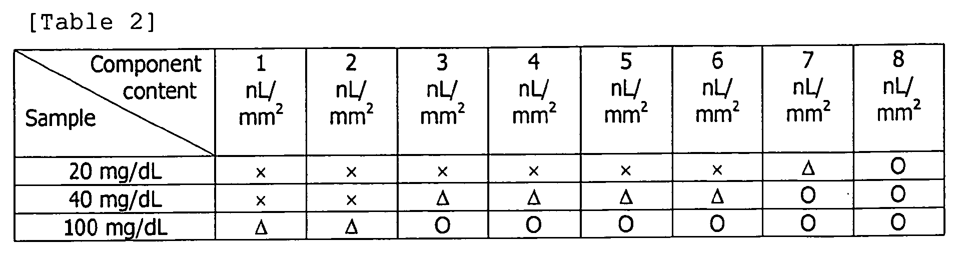

As is clear from Table 2, in one analytical test piece (one analytical test piece which is configured such that the detection component content of the reaction sites is continuously increased from one end of the reaction section to another end thereof), the cholesterol content is measured in an analog manner. In both Examples 1 and 2, the analytical test piece (evaluation sample) was produced through the ink-jet method. However, the analytical test piece may be produced through a technique appropriately selected from among, for example, screen printing and liquid impregnation.

### Industrial Applicability

The analytical test piece of the present invention is effectively employed for the production of a test chip or the like for testing or analyzing properties of a sample containing an analyte (e.g., a body fluid (in particular, urine or blood) of a human or an animal) in the fields of research, drug development, diagnosis, medicine, etc.

## Claims

1. An analytical test piece comprising a carrier, and a reaction section containing a detection component, the reaction section being provided on the surface and/or in the interior of the carrier, wherein in use, when a sample containing an analyte is introduced to the surface of the carrier, the analyte comes into contact and reacts with the detection component contained in the reaction section, to thereby generate a detectable substance (signal substance) or to exhibit a detectable property (signal property), and
wherein the amount of the detection component contained in the reaction section is continuously increased or decreased from one end of the reaction section to another end thereof.

2. An analytical test piece as described in claim 1,
wherein the detection component contained in the reaction section is formed of a plurality of species which are isolated or separated from one another on the reaction section, and the amount of each of the detection component species is continuously increased or decreased from one end of the reaction section to another end thereof.

3. An analytical test piece as described in claim 1 or 2,
wherein the detection component contained in the reaction section is in the form of an aggregate of spots (dots) which are provided in a predetermined arrangement pattern (spot pitch).

4. An analytical test piece as described in claim 3,
wherein the amount of the detection component contained in the spots (dots) constituting the arrangement pattern is continuously increased or decreased from one end of the reaction section to another end thereof.

5. An analytical test piece as described in claim 3 or 4,
wherein the spots (dots) constituting the arrangement pattern have an arrangement density which is continuously increased or decreased from one end of the reaction section to another end thereof.

6. An analytical test piece as described in any of claims 3 to 5, wherein the size of the spots (dots) constituting the arrangement pattern is continuously increased or decreased from one end of the reaction section to another end thereof.

7. An analytical test piece as described in any of claims 3 to 6, wherein the amount of the detection component contained in the spots (dots) constituting the arrangement pattern, the amount being measured in a thickness direction of the carrier, is continuously increased or decreased from one end of the reaction section to another end thereof.

8. An analytical test piece comprising a carrier, and a reaction section containing a detection component, the reaction section being provided on the surface and/or in the interior of the carrier, wherein in use, when a sample containing an analyte is introduced to the surface of the carrier, the analyte comes into contact and reacts with the detection component contained in the reaction section, to thereby generate a detectable substance (signal substance) or to exhibit a detectable property (signal property), and
wherein the reaction section is formed of a plurality of divided reaction sites, and the amount of the detection component contained in each reaction site of the reaction section is increased or decreased from one end of the reaction section to another end thereof in a stepwise manner when the reaction sites are adjacent to one another, or in a fragmentary manner when the reaction sites are isolated or separated from one another.

9. An analytical test piece as described in claim 8,
wherein the detection component contained in the reaction section is formed of a plurality of species which are isolated or separated from one another on the reaction section; and the amount of each of the detection component species, which respectively correspond to a plurality of the reaction sites, is increased or decreased from one end of the reaction section to another end thereof in a stepwise manner when the reaction sites are adjacent to one another, or in a fragmentary manner when the reaction sites are isolated or separated from one another.

10. An analytical test piece as described in claim 8 or 9,
wherein the detection component contained in the reaction section is in the form of an aggregate of spots (dots) which are provided in a predetermined arrangement pattern (spot pitch).

11. An analytical test piece as described in claim 10,
wherein the amount of the detection component contained in the spots (dots) constituting the arrangement pattern is increased or decreased, in a plurality of the divided reaction sites constituting the reaction section, from one end of the reaction section to another end thereof in a continuous, stepwise manner or in a discontinuous, fragmentary manner.

12. An analytical test piece as described in claim 10 or 11,
wherein the spots (dots) constituting the arrangement pattern have an arrangement density which is increased or decreased, in a plurality of the divided reaction sites constituting the reaction section, from one end of the reaction section to another end thereof in a continuous, stepwise manner or in a discontinuous, fragmentary manner.

13. An analytical test piece as described in any of claims 10 to 12, wherein the size of the spots (dots) constituting the arrangement pattern is increased or decreased, in a plurality of the divided reaction sites constituting the reaction section, from one end of the reaction section to another end thereof in a continuous, stepwise manner or in a discontinuous, fragmentary manner.

14. An analytical test piece as described in any of claims 10 to 13, wherein the amount of the detection component contained in the spots (dots) constituting the arrangement pattern, the amount being measured in a thickness direction of the carrier, is increased or decreased, in a plurality of the divided reaction sites constituting the reaction section, from one end of the reaction section to another end thereof in a continuous, stepwise manner or in a discontinuous, fragmentary manner.

15. An analytical test piece as described in any of claims 1 to 14, wherein the carrier is a fibrous carrier or a porous carrier.

16. An analytical test piece as described in any of claims 1 to 15, wherein the reaction section containing the detection component is provided on the surface and/or in the interior of the carrier through the ink-jet method.
